# EUROPEAN PATENT SPECIFICATION

(11) **EP 4 154 885 B1**
(45) Date of publication and mention of the grant of the patent: **06.08.2025**
(21) Application number: 21808425.9
(22) Date of filing: 18.05.2021
(51) Int. Cl.: A61K 31/473, A61K 9/06, A61K 9/08, A61K 9/70, A61K 47/10, A61K 47/12, A61P 25/16, A61K 47/14

(54) **APOMORPHINE-CONTAINING PERCUTANEOUS ABSORPTION TYPE PREPARATION**
APOMORPHIN ENTHALTENDE ZUBEREITUNG VOM PERKUTANEN ABSORPTIONSTYP
PRÉPARATION DE TYPE À ABSORPTION PERCUTANÉE CONTENANT DE L'APOMORPHINE

(30) Priority: 20.05.2020 JP 2020087793
(43) Date of publication of application: 29.03.2023
(73) Proprietor: MEDRx Co., Ltd., Higashikagawa-shi Kagawa 769-2712 (JP)
(72) Inventor: YAMASAKI, Keiko, Higashikagawa-shi Kagawa 769-2712 (JP); TAKEMOTO, Makiko, Higashikagawa-shi Kagawa 769-2712 (JP); NAGAO,Yuko, Higashikagawa-shi Kagawa 769-2712 (JP); KONDO,Shunya, Higashikagawa-shi Kagawa 769-2712 (JP)
(74) Representative: Kraus & Lederer PartGmbB
(86) International application number: PCT/JP2021/018793
(87) International publication number: WO 2021/235436

(56) References cited:
- WO-A1-96/19956
- JP-A- 2002 087 954
- JP-A- 2011 511 088
- JP-A- 2013 213 035
- JP-A- 2015 151 380
- US-A1- 2011 150 946

## Description

### TECHNICAL FIELD

### (Cross-Reference to Related Applications)

This application claims the benefit of Japanese Patent Application No. 2020-87793, filed with the Japan Patent Office on May 20, 2020.

The present invention relates to a transdermal formulation containing apomorphine or a salt thereof as an active ingredient. In particular, it relates to a transdermal composition containing a high concentration of apomorphine or a salt thereof and being capable of administering a sufficient amount of apomorphine transdermally for treatment.

### BACKGROUND OF THE INVENTION

Apomorphine is a dopamine D1- and D2-like receptor agonist that stimulates these receptors in the striatum, resulting in rapid improvement of off-symptoms in Parkinson's disease. In particular, subcutaneous injections are used clinically as a rescue drug when off-symptoms occur due to lack of L-DOPA efficacy. For example, Patent Document 1 proposes a pharmaceutical composition used as an injection comprising 1) apomorphine as an active ingredient, 2) a water-soluble co-solvent, 3) an antioxidant, and 4) water, with a pH greater than 4.

However, many patients experience off-symptoms due to lack of L-DOPA efficacy not only during the day, but also at night or early in the morning when drug administration is difficult. That causes interference with sleep and the movement at the time of waking up.

Among the prior art, related transdermal formulations are described in Patent Documents 2 and 3.

Therefore, there is a need for a transdermal formulation that can be applied before bedtime to improve off-symptoms that occur at night or early in the morning, and that allows continuous administration of apomorphine from bedtime to early morning.

For transdermal formulations for nighttime and early morning off-symptom rescue, rapid and sustained skin permeation of apomorphine is required. In particular, a high concentration of the drug dissolved in the formulation is fundamental to achieve fast skin permeation after application. However, until now, no method has been developed to dissolve apomorphine in sufficient concentration.

### PRIOR ART

### PATENT DOCUMENT

Patent Document 1: JP 2017-81947 A
Patent Document 2: US 2011/150946 A1
Patent Document 3: JP 2002-087954 A

### SUMMARY OF THE INVENTION

### PROBLEM TO BE SOLVED BY THE INVENTION

The present invention aims to provide a transdermal formulation in which apomorphine or a salt thereof is dissolved at a high concentration, so that a sufficient amount of apomorphine for treatment can be absorbed in a short time, and can be administered continuously.

### MEANS FOR SOLVING THE PROBLEM

As a result of intensive studies to solve the above problem, the inventors found that a solution containing a high concentration of apomorphine or a salt thereof can be prepared by combining a specific polyhydric alcohol and propylene carbonate. They then succeeded in creating a formulation that allows rapid and continuous transdermal administration of a sufficient amount of apomorphine using this highly concentrated solution containing apomorphine or a salt thereof, and completed the present invention which provides a transdermal formulation according to claim 1, a patch according to claim 13, as well as the use thereof according to claim 14.

### EFFECT OF THE INVENTION

According to the present invention, it is possible to prepare a solution containing apomorphine in high concentration, and to provide a transdermal formulation that allows a sufficient amount of apomorphine to be absorbed into the body through the skin in a short time and administered continuously.

### BRIEF DESCRIPTION OF THE DRAWING

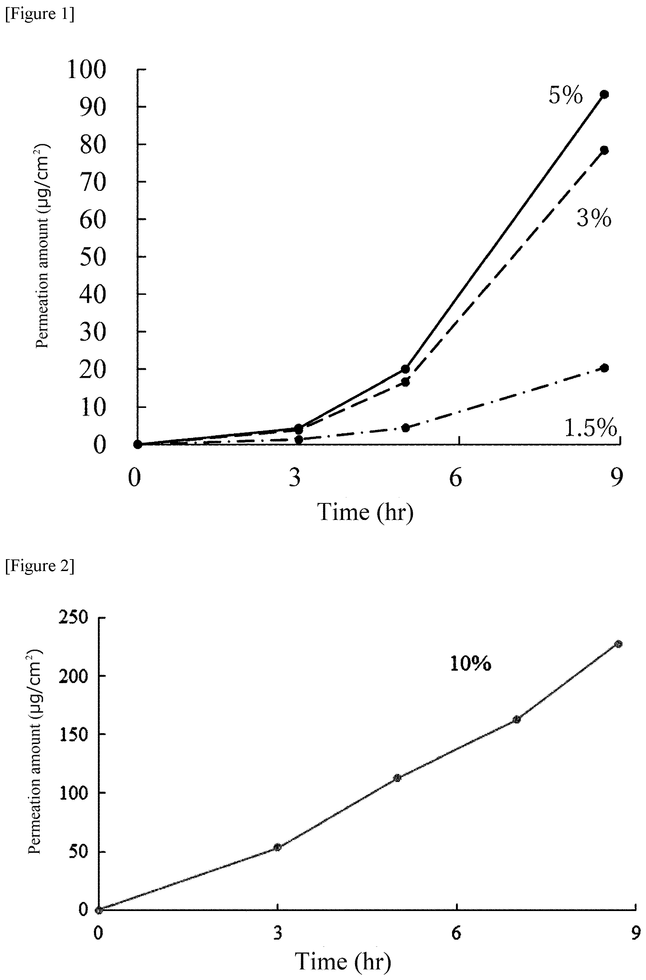

Figure 1 shows the result of in vitro skin permeability study of apomorphine hydrochloride using a Franz cell for the formulations of Examples 1 to 3. The vertical axis represents the amount of permeation (µg/cm²) and the horizontal axis represents the elapsed time (hours). The numbers (1.5, 3, and 5) in the graph represent the content of apomorphine hydrochloride in the formulation (weight %).
Figure 2 shows the result of in vitro skin permeability study of apomorphine hydrochloride using a Franz cell for the formulation of Example 4. The vertical axis represents the amount of permeation (µg/cm²) and the horizontal axis represents the elapsed time (hours). The number (10) in the graph represents the content of apomorphine hydrochloride in the formulation (weight %).

### EMBODIMENT FOR CARRYING OUT THE PRESENT INVENTION

The transdermal formulation of the present invention, in one embodiment, contains apomorphine or a salt thereof, a polyhydric alcohol with a carbon number of 6 or less and/or a low molecular weight polyethylene glycol, and propylene carbonate. A solution containing this combination can dissolve apomorphine or a salt thereof at a concentration as high as about 5% by weight or more, or about 10% by weight. Even in hydrophilic preparations prepared by adding other ingredients to this highly concentrated solution, it is possible to maintain the concentration of apomorphine or a salt thereof at a sufficiently high level. In this way, a transdermal formulation containing apomorphine or a salt thereof with rapid and sustained skin permeation can be obtained.

The transdermal formulation of the present invention contains apomorphine or a salt thereof as an active ingredient. The content of apomorphine or a salt thereof in the transdermal formulation of the present invention can be, for example, about 2% by weight or more, about 3% by weight or more, or about 4% by weight or more. The upper limit of the content of apomorphine or a salt thereof can be, for example, about 15% by weight, about 12% by weight, or about 10% by weight. In one embodiment, the transdermal formulation of the present invention contains no active ingredient other than apomorphine or a salt thereof.

In one embodiment, apomorphine or a salt thereof is, for example, free apomorphine, a salt of apomorphine and an organic acid, or a salt of apomorphine and an inorganic acid, etc. Apomorphine hydrochloride may also be used. Apomorphine or a salt thereof may also be a hydrate or a solvate of free apomorphine or a salt thereof.

In one embodiment, the transdermal formulation of the present invention can contain either a polyhydric alcohol with a carbon number of 6 or less, or a low molecular weight polyethylene glycol alone or both.

The amount of the polyhydric alcohol with a carbon number of 6 or less and the low molecular weight polyethylene glycol is not particularly limited as long as a sufficient amount of apomorphine or a salt thereof can be dissolved, but in total can be, for example, about 20% or more by weight, about 30% or more by weight, about 40% or more by weight, about 50% or more by weight, or about 60% or more by weight, and can be about 90% or less by weight, about 85% or less by weight, or about 80% or less by weight of the transdermal formulation. With regard to the amount of the polyhydric alcohol with a carbon number of 6 or less and the low molecular weight polyethylene glycol, the fewer is the better from the viewpoint of skin permeability and the more is the better from the viewpoint of skin irritation.

Herein, the polyhydric alcohol with a carbon number of 6 or less (hereinafter referred to as "said polyhydric alcohol") is, for example, an alcohol having 2 to 8 or about 6 hydroxyl groups (OH), and is not restricted to such polyhydric alcohols. Preferred said polyhydric alcohol can include divalent alcohols and trivalent alcohols. The carbon number of said polyhydric alcohol may be, for example, 5 or less, or 3 or less. Specifically, said polyhydric alcohol can include, for example, propylene glycol, 1,3-propanediol, 1,3-butanediol, 1,4-butanediol, pentanediol, hexanediol, and glycerin. Of these, propylene glycol, 1,3-butanediol, and glycerin are preferred. The transdermal formulation of the present invention may contain one of these, or any two or more.

In the case that said polyhydric alcohol is glycerin (99% or more), its content can be, for example, in the range of about 1% to about 50% by weight, about 5% to about 40% by weight, or about 10% to about 30% by weight, of the transdermal formulation. In the case that said polyhydric alcohol is 1,3-butanediol, its content can be, for example, in the range of about 0.5% to about 30% by weight, or about 1% to about 25% by weight, of the transdermal formulation. In the case that said polyhydric alcohol is propylene glycol, its content can be, for example, in the range of about 1 to about 60% by weight, or about 3% to about 55% by weight, of the transdermal formulation.

The low molecular weight polyethylene glycol as claimed are polyethylene glycols with a molecular weight of about 200 to about 600, and polyethylene glycols with a molecular weight of about 200 to about 300 may be used.

The amount of the propylene carbonate can be, for example, in the range of about 1% to about 40% by weight, about 2% to about 30% by weight, or about 5% to about 25% by weight, of the transdermal formulation.

With regard to the weight ratio of said polyhydric alcohol and/or low molecular weight polyethylene glycol to propylene carbonate, for example, when propylene carbonate is taken as 1, the total of said polyhydric alcohol and low molecular weight polyethylene glycol can be in the range of about 1 to about 10, about 2 to about 9, or about 3 to about 8.

In one embodiment, the transdermal formulation of the present invention can contain an antioxidant. By adding an antioxidant, apomorphine or a salt thereof can be stored stably without coloration. The antioxidant can include tocopherol acetate, sodium edetate, erythorbic acid, 1,3-butylene glycol, dibutyl hydroxytoluene, ascorbic acid, propyl gallate, sodium sulfite and sodium pyrosulfite.

The content of the antioxidant is not particularly limited as long as the stability of apomorphine or a salt thereof is obtained, and can be in the range of about 0.01% to about 0.5% by weight, about 0.02% to about 0.3% by weight, or about 0.05% to about 0.2% by weight.

In one embodiment, the transdermal formulation of the present invention can contain an organic acid. The addition of the organic acid can improve the skin permeability of apomorphine or a salt thereof. The kind of the organic acid is not particularly limited, but can include, for example, oleic acid, isostearic acid, capric acid, sorbic acid, levulinic acid, lauric acid, myristic acid, palmitic acid, and stearic acid.

The content of the organic acid is not particularly limited as long as sufficient skin permeability of apomorphine or a salt thereof can be obtained, and may be in the range of about 0.1% to about 5% by weight, about 0.2% to about 4% by weight, or about 0.3% to about 3% by weight, of the transdermal formulation.

In one embodiment, the transdermal formulation of the present invention can contain water. This can reduce skin irritation. The amount of water can be, for example, in the range of about 0.5% to about 30% by weight, about 1% to about 20% by weight, or about 2% to about 10% by weight, of the transdermal formulation.

In one embodiment, the transdermal formulation of the present invention can contain an emulsifier. By adding an emulsifier, when the transdermal formulation contains water, substances with low solubility in water, such as propylene carbonate and organic acids, can be dispersed stably in the liquid. Such emulsifiers can include, for example, natural emulsifiers such as gum arabic, gelatin, tragacanth, lecithin (phosphatidylcholine), and cholesterol; anionic surfactants such as soap and sodium alkyl sulfate; polyoxyethylene sorbitan fatty acid esters such as monooleoyl polyoxyethylene sorbitan; glycerol fatty acid esters such as polyoxyethylene castor oil derivatives, polyoxyethylene hardened castor oil, glycerol monostearate and sorbitan monooleate; sorbitan fatty acid esters such as sorbitan monostearate and sorbitan sesquiolate; polyoxyethylene higher alcohol ethers such as polyoxyethylene cetyl ether; nonionic surfactants such as polyoxyethylene alkyl phenol, and polyoxyethylene oxypropylene copolymers (e.g., Pluronic (registered trademark)); cationic surfactants such as cetyltrimethylammonium chloride; amphoteric surfactants, etc.

In one embodiment, the transdermal formulation of the present invention can contain an alkanolamine. The alkanolamine can be, for example, a primary, secondary, or tertiary alkanolamine with carbon numbers from 2 to 12. Specifically, the alkanolamine can be, for example, diethanolamine, triethanolamine, diisopropanolamine, and triisopropanolamine. The content of the alkanolamine can be, for example, in the range of about 0.005% to about 0.4% by weight, or about 0.01% to about 0.3% by weight, of the transdermal formulation.

The transdermal formulations of the present invention can also contain various additives used in topical or cosmetic formulations as needed. Such additives can include fragrances, antioxidants, preservatives, colorants, buffers, pH adjusters, UV absorbers, antibacterial agents, etc. The preservatives can include sorbic acid, taurine, etc. The pH adjusters can include organic acids such as citric acid, acetic acid, tartaric acid, etc.; and inorganic acids such as phosphoric acid, hydrochloric acid, etc.

The final dosage form of the transdermal formulation of the present invention is not particularly limited, and can be, for example, a liquid, gel, ointment, or a carrier impregnated with at least one of them. The transdermal formulation of the present invention is prepared into various external dosage forms for the absorption of a drug through the skin and applied to the skin by application or spray. The transdermal formulation of the present invention can also be applied by applying a carrier (nonwoven fabric, foaming matrix, etc.) impregnated with a liquid or gel formulation, for example, to the skin, which facilitates dose adjustment and allows ease of handling.

The transdermal formulation of the present invention can be used as any pharmaceutical product comprising apomorphine or a salt thereof as an active ingredient. Since the transdermal formulation of the present invention achieves a rapid absorption and a sustained effect, it is used, for example, as an off-symptom remedy for Parkinson's disease.

### EXAMPLE

Hereinafter, the present invention will be described with reference to Examples.

### 1. Study of the solubility of apomorphine hydrochloride

The solubility of apomorphine hydrochloride in various solvents was investigated. The results are shown in Table 1.

When used alone, water, 1,3-butanediol, polyethylene glycol, propylene glycol, glycerin, and propylene carbonate all had a solubility of 3% by weight or less for apomorphine hydrochloride. Especially, solubility in propylene carbonate was extremely low at 0.25% by weight.

On the other hand, when a low molecular weight polyethylene glycol was combined with propylene carbonate, an apomorphine hydrochloride solution of 8% by weight was able to be prepared. Furthermore, in a combination of propylene glycol and propylene carbonate, using a solvent in which the amount by weight of propylene glycol was five times or more as large as that of propylene carbonate allowed an apomorphine hydrochloride solution of 10% by weight to be prepared.

Likewise, using a solvent in which either of 1,3-butanediol or concentrated glycerin (99%) was mixed with propylene carbonate at a weight ratio of 5:1 also allowed an apomorphine hydrochloride solution of 10% by weight to be prepared.

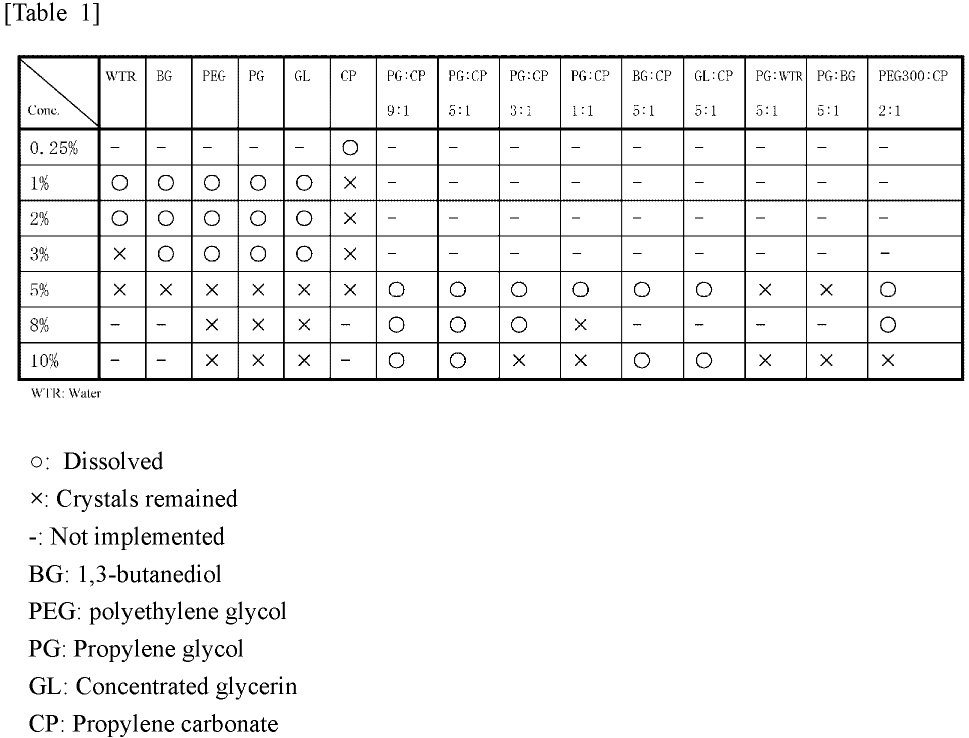

### 2. Preparation of apomorphine hydrochloride solution

In view of the above study 1, prepared were a 1.5% apomorphine hydrochloride liquid formulation (Example 1), a 3% apomorphine hydrochloride liquid formulation (Example 2), and a 5% apomorphine hydrochloride liquid formulation (Example 3), wherein the formulations contained said polyhydric alcohol (propylene glycol, concentrated glycerin, and 1,3-butanediol) and propylene carbonate at a weight ratio of about 3.7:1 to about 3.5:1 (said polyhydric alcohol: propylene carbonate). Also prepared was a 10% apomorphine hydrochloride liquid formulation (Example 4), which contained a mixture of said polyhydric alcohol and polyethylene glycol, and propylene carbonate at a weight ratio of about 7.9:1 (the mixture : propylene carbonate). Each composition (weight %) is shown in Table 2. In all cases, apomorphine hydrochloride was completely dissolved.

**[Table 2]**

| | 1.5% Example 1 | 3% Example 2 | 5% Example 3 | 10% Example 4 |
|---|---|---|---|---|
| Apomorphine hydrochloride | 1.5 | 3 | 5 | 10 |
| Purified water | 4 | 4 | 4 | - |
| Propylene glycol | 48.54 | 47.03 | 45.03 | 5 |
| Conc. glycerin | 22 | 22 | 22 | 26.97 |
| 1,3-Butanediol | 3 | 3 | 3 | 22 |
| PEG300 | - | - | - | 25 |
| Propylene carbonate | 20 | 20 | 20 | 10 |
| Isostearic acid | 0.7 | 0.7 | 0.7 | - |
| Oleic acid | - | - | - | 0.7 |
| Triethanolamine | 0.02 | 0.03 | 0.03 | 0.06 |
| Sodium pyrosulfite | 0.12 | 0.12 | 0.12 | 0.15 |
| Phosphatidylcholine | 0.12 | 0.12 | 0.12 | 0.12 |
| Total | 100 | 100 | 100 | 100 |

### [Skin Permeability Test]

In vitro skin permeability test was conducted on the liquid formulations of Examples 1 to 3 (transdermal absorption preparation of the present invention) impregnated in a carrier (a patch) using a Franz cell. The skin used for the test was the abdominal skin of a 5-week-old male hairless rat. The cumulative amount of skin permeation (µg/cm²) of apomorphine hydrochloride during 9 hours after the start of the test is shown in Figure 1.

All of the liquid formulations showed a sustained increase in skin permeation, but those containing 3% or more by weight of apomorphine hydrochloride showed a faster onset of transdermal absorption, suggesting a shorter time for the drug effect to be achieved.

This suggests that the apomorphine hydrochloride concentration in the formulation is preferably at least 3% by weight to achieve skin permeability early after administration.

In vitro skin permeability test was conducted on the liquid formulation of Example 4 (transdermal absorption formulation of the present invention) impregnated in a carrier (a patch) using a Franz cell. The skin used for the test was human skin. The cumulative amount of skin permeation (µg/cm²) of apomorphine hydrochloride during 9 hours after the start of the test is shown in Figure 2.

It was suggested that those containing 10% by weight of apomorphine hydrochloride had a faster onset of transdermal absorption and a shorter time for the drug effect to be achieved even in human skin.

### [Skin irritation test]

The liquid formulation obtained in Example 3 (transdermal absorption formulation of the present invention) impregnated in a carrier (a patch) was applied to the skin surface of a rabbit for 24 hours. The skin irritancy after peeling was evaluated according to Draize's criteria. As a result, the skin irritation index P.I.I. was 0.1, indicating that the formulation had sufficiently low skin irritation.

### [Stability test]

Table 3 shows the results after the liquid formulation obtained in Example 3 (transdermal absorption formulation of the present invention) impregnated in a carrier (a patch) was sealed and filled into an aluminum laminate container and stored in a constant temperature oven at 25°C for 6 months. Apomorphine hydrochloride is known to be unstable and easily discolored, but the transdermal absorption formulation or patch of the present invention was confirmed to be stable.

**[Table 3]**

| | Initial | After 6-month storage at 25°C |
|---|---|---|
| Properties (color tone) | colorless | Colorless to slightly yellow |
| Content of apomorphine hydrochloride (vs initial value) | 100% | 98.2% |

## Claims

1. A transdermal formulation, comprising apomorphine or a salt thereof, a polyhydric alcohol with a carbon number of 6 or less and/or a low molecular weight polyethylene glycol, and propylene carbonate, wherein the low molecular weight polyethylene glycol is polyethylene glycol with a molecular weight of about 200 to about 600.

2. The transdermal formulation according to Claim 1, containing 2% or more by weight of said apomorphine or a salt thereof.

3. The transdermal formulation according to Claim 1, containing 4% or more by weight of said apomorphine or a salt thereof.

4. The transdermal formulation according to any one of Claims 1 to 3, containing from 5% to 40% by weight of said propylene carbonate.

5. The transdermal formulation according to any one of Claims 1 to 4, wherein the total weight of the polyhydric alcohol and the low molecular weight polyethylene glycol is in the range of 1 to 10, when the weight of propylene carbonate is taken as 1.

6. The transdermal formulation according to Claim 5, wherein the polyhydric alcohol is one or more selected from the group consisting of propylene glycol, glycerin, and 1,3-butanediol.

7. The transdermal formulation according to any one of Claims 1 to 6, further comprising an antioxidant.

8. The transdermal formulation according to Claim 7, wherein the antioxidant is sodium pyrosulfite.

9. The transdermal formulation according to any one of Claims 1 to 8, further comprising an organic acid or water.

10. The transdermal formulation according to Claim 9, wherein the organic acid is isostearic acid or oleic acid.

11. The transdermal formulation according to any one of Claims 1 to 10, further comprising an emulsifier.

12. The transdermal formulation according to any one of Claims 1 to 11, wherein the dosage form is a liquid, gel, or ointment.

13. A patch, comprising a carrier impregnated with the transdermal formulation according to Claim 12.

14. The transdermal formulation according to any one of Claims 1 to 12 or the patch according to Claim 13, for use in remedying off-symptoms of Parkinson's disease.

## Patentansprüche

1. Transdermale Formulierung, umfassend Apomorphin oder ein Salz davon, einen mehrwertigen Alkohol mit einer Kohlenstoffzahl von 6 oder weniger und/oder ein Polyethylenglykol mit niedrigem Molekulargewicht und Propylencarbonat, wobei das Polyethylenglykol mit niedrigem Molekulargewicht ein Polyethylenglykol mit einem Molekulargewicht von etwa 200 bis etwa 600 ist.

2. Transdermale Formulierung nach Anspruch 1, enthaltend 2 Gew.-% oder mehr des Apomorphins oder eines Salzes davon.

3. Transdermale Formulierung nach Anspruch 1, enthaltend 4 Gew.-% oder mehr des Apomorphins oder eines Salzes davon.

4. Transdermale Formulierung nach einem der Ansprüche 1 bis 3, die 5 bis 40 Gew.-% des Propylencarbonats enthält.

5. Transdermale Formulierung nach einem der Ansprüche 1 bis 4, wobei das Gesamtgewicht des mehrwertigen Alkohols und des niedermolekularen Polyethylenglykols im Bereich von 1 bis 10 liegt, wenn das Gewicht des Propylencarbonats als 1 angenommen wird.

6. Transdermale Formulierung nach Anspruch 5, wobei der mehrwertige Alkohol ein oder mehrere ausgewählt aus der Gruppe bestehend aus Propylenglykol, Glycerin und 1,3-Butandiol ist.

7. Transdermale Formulierung nach einem der Ansprüche 1 bis 6, die außerdem ein Antioxidans enthält.

8. Transdermale Formulierung nach Anspruch 7, wobei das Antioxidans Natriumpyrosulfit ist.

9. Transdermale Formulierung nach einem der Ansprüche 1 bis 8, die außerdem eine organische Säure oder Wasser enthält.

10. Transdermale Formulierung nach Anspruch 9, wobei die organische Säure Isostearinsäure oder Ölsäure ist.

11. Transdermale Formulierung nach einem der Ansprüche 1 bis 10, die außerdem einen Emulgator enthält.

12. Transdermale Formulierung nach einem der Ansprüche 1 bis 11, wobei die Darreichungsform eine Flüssigkeit, ein Gel oder eine Salbe ist.

13. Pflaster, umfassend einen Träger, der mit der transdermalen Formulierung nach Anspruch 12 imprägniert ist.

14. Transdermale Formulierung nach einem der Ansprüche 1 bis 12 oder Pflaster nach Anspruch 13, zur Verwendung bei der Behebung von Off-Symptomen der Parkinson-Krankheit.

## Revendications

1. Formulation transdermique comprenant de l'apomorphine ou un de ses sels, un alcool polyhydrique avec un nombre de carbone inférieur ou égal à 6 et/ou un polyéthylène glycol de faible poids moléculaire, et du carbonate de propylène, le polyéthylène glycol de faible poids moléculaire étant un polyéthylène glycol dont le poids moléculaire est compris entre 200 et 600 environ.

2. Formulation transdermique selon la revendication 1, contenant 2% ou plus en poids de ladite apomorphine ou d'un de ses sels.

3. Formulation transdermique selon la revendication 1, contenant 4% ou plus en poids de ladite apomorphine ou d'un de ses sels.

4. Formulation transdermique selon l'une des revendications 1 à 3, contenant de 5% à 40% en poids de carbonate de propylène.

5. Formulation transdermique selon l'une des revendications 1 à 4, dans laquelle le poids total de l'alcool polyhydrique et du polyéthylène glycol de faible poids moléculaire est compris entre 1 et 10, lorsque le poids du carbonate de propylène est égal à 1.

6. Formulation transdermique selon la revendication 5, dans laquelle l'alcool polyhydrique est un ou plusieurs alcools choisis dans le groupe constitué par le propylène glycol, la glycérine et le 1,3-butanediol.

7. Formulation transdermique selon l'une des revendications 1 à 6, comprenant en outre un antioxydant.

8. Formulation transdermique selon la revendication 7, dans laquelle l'antioxydant est le pyrosulfite de sodium.

9. Formulation transdermique selon l'une des revendications 1 à 8, comprenant en outre un acide organique ou de l'eau.

10. Formulation transdermique selon la revendication 9, dans laquelle l'acide organique est l'acide isostéarique ou l'acide oléique.

11. Formulation transdermique selon l'une des revendications 1 à 10, comprenant en outre un émulsifiant.

12. Formulation transdermique selon l'une des revendications 1 à 11, dans laquelle la forme de dosage est un liquide, un gel ou une pommade.

13. Patch, comprenant un support imprégné de la formulation transdermique selon la revendication 12.

14. Formulation transdermique selon l'une des revendications 1 à 12 ou patch selon la revendication 13, pour une utilisation dans le traitement des symptômes de phase off de la maladie de Parkinson.
